(19) Europäisches Patentamt

European Patent Office

Office européen des brevets

(11) **EP 2 143 701 B1**

(12) **EUROPEAN PATENT SPECIFICATION**

(45) Date of publication and mention
of the grant of the patent:
**02.03.2011 Bulletin 2011/09**

(51) Int Cl.:
**C07C 5/333** (2006.01)      **C07C 11/06** (2006.01)

(21) Application number: **09155574.8**

(22) Date of filing: **19.03.2009**

(54) **Hybrid autothermal catalytic process for converting alkanes to alkenes and catalysts useful for same**

Autothermisches katalytisches Hybrid-Verfahren zur Umwandlung von Alkanen in Alkene und Katalysatoren dafür

Procédé catalytique thermique automatique hybride pour la conversion d'alcanes en alcènes et catalyseurs utiles pour ledit procédé

(84) Designated Contracting States:
**DE FR GB**

(30) Priority: **10.07.2008 US 134496 P**

(43) Date of publication of application:
**13.01.2010 Bulletin 2010/02**

(73) Proprietor: **Rohm and Haas Company
Philadelphia, PA 19106-2399 (US)**

(72) Inventors:
• **Han, Scott
Lawrenceville, NJ 08648 (US)**
• **Ruettinger, Wolfgang
East Windsor, NJ 08520 (US)**

(74) Representative: **Kent, Venetia Katherine
Patent Outsourcing Limited
1 King Street
Bakewell
Derbyshire DE45 1DZ (GB)**

(56) References cited:
**EP-A- 0 568 303     EP-A- 1 916 230
WO-A-02/26668     US-A- 5 430 209**

• **DATABASE WPI Week 200455 Thomson Scientific, London, GB; AN 2004-562610 XP002548653 & CN 1 472 005 A (UNIV NANJING) 4 February 2004 (2004-02-04) & CN 1 472 005 A (UNIV NANJING [CN]) 4 February 2004 (2004-02-04)**

**Description**

Field of the Invention

The present invention relates to thermally integrated processes for conversion of alkanes to their corresponding alkenes, which may then be further converted by catalytic partial oxidation to oxidation products, including unsaturated carboxylic acids and unsaturated nitriles.

Background of the Invention

Well-known commercial processes for the production of monomers, such as unsaturated carboxylic acids and unsaturated nitriles, typically start with one or more alkenes and convert them, by catalytic vapor phase oxidation, to the desired monomer products. In view of the pressures exerted by competition in the industry, and the price difference between alkanes and their corresponding alkenes, such as propane and propene, respectively, efforts are being made to develop processes in which an alkane is used as the starting material to, ultimately, produce the desired monomers at a lower overall cost.

One well-known alternative is to simply add an upstream reaction stage to the process, in which an alkane is first converted to the corresponding alkene, in the presence of a suitable catalyst. The resulting alkene (e.g., propene) product is then fed to the customary oxidation reaction stages, for oxidation of the alkene (e.g., first to acrolein and then to the desired monomer product, as in the two-step oxidation of propene to form acrylic acid). For example, both European Patent Application No. EP0117146 and US Patent No. 5,705,684 describe multi-stage catalytic processes for converting an alkane (propane) to the corresponding unsaturated carboxylic acid (acrylic acid) which includes an initial alkane-to-alkene conversion stage having one or more suitable catalysts to produces a product stream comprising alkene, which is fed to one or more downstream oxidation stages.

Various catalysts and methods are known to catalyze conversion of alkanes to their corresponding alkenes. However, catalysts which catalyze the exothermic conversion of an alkane to its corresponding alkene also produce heat. This heat must be removed from the product stream or otherwise integrated or managed prior to sending the exothermic conversion product stream containing the desired alkene to downstream processes for conversion to the desired oxidation products (e.g., unsaturated carboxylic acids and unsaturated nitriles).

There are also catalysts which are known to catalyze the non-oxidative dehydrogenation of an alkane, in the presence of a "weak" oxidant, such as steam or carbon dioxide, to form the corresponding alkene without production of excess heat. Some non-oxidative dehydrogenation catalysts perform better in the absence of oxygen, while others tolerate the presence of minor amounts of oxygen, along with the weak oxidant, without significant loss of activity.

Non-oxidative dehydrogenation reactions are endothermic and, therefore, require addition of heat to the process. One way of providing heat to the non-oxidative dehydrogenation process is to recover heat from another, separate, process, or even from a related downstream process, such as a process step in which the alkene produced by oxidative dehydrogenation is utilized, and recycle that heat back to the non-oxidative dehydrogenation reaction zone.

Provision of heat to non-oxidative dehydrogenation processes has also been achieved by creating heat in a preceding, upstream oxidation or combustion step which consumes a fuel such as hydrogen or a hydrocarbon, and oxygen, and then conveying that heat to the non-oxidative dehydrogenation process. For example, a portion of the alkane to be dehydrogenated may, itself, be used as the hydrocarbon fuel and burned (combusted/oxidized) with oxygen in a preceding upstream reaction zone to produce the required heat. More particularly, a portion of the alkane to be dehydrogenated may be combusted in the presence of oxygen and a suitable combustion catalyst, to produce a heated stream containing the products of combustion (i.e., carbon oxides and water), unconsumed oxygen and unconsumed alkane. The heated stream can then be fed directly to a non-oxidative catalytic dehydrogenation reaction stage where the unreacted alkane is converted to the corresponding alkene in the presence of a suitable non-oxidative dehydrogenation catalyst. However, even while such methods avoid the need to use hydrocarbons different from the alkane to be converted, they require consumption of a portion of the alkane, which leaves less available for conversion to the desired product in the non-oxidative dehydrogenation stage. Furthermore, products of combustion are typically incidentally formed, which increases the amount of unwanted by-products, without any contribution to the quantity of the desired alkene product. Thus, when a portion of the alkane reactant itself is burned, a diminished amount of alkane remains available for the non-oxidative dehydrogenation reaction and less of the desired alkene product is produced.

Integrated processes have also been developed wherein the production of heat is accomplished in the non-oxidative dehydrogenation reaction zone itself, thereby reducing the amount of equipment and capital investment required. In other words, a fuel different than the alkane to be dehydrogenated, is burned (i.e., oxidized or combusted) with oxygen in the non-oxidative dehydrogenation reaction zone to provide the necessary heat. For example, U.S. Patent No. 7,291,761 describes an autothermal process for the catalytic dehydrogenation of a $C_2$-$C_8$ alkane, in the presence of a dehydrogenation catalyst and molecular oxygen, to produce the corresponding $C_2$-$C_8$ alkene. The product gas which exits the non-oxidative dehydrogenation reaction zone is divided into two substreams of identical composition, one of which is recycled to the reaction zone to provide a continuous source of hydrogen fuel for oxidation (burning) to provide the heat required for the ongoing catalytic non-oxidative dehydrogenation reaction. Suitable non-oxidative dehydrogentation catalysts are described as metals/metal oxides (e.g., chromium oxide and/or Pt/aluminum oxide). Molecular hydrogen and at least one dehydrogenated hydrocarbon (e.g., a $C_2$-$C_8$ alkene) are formed in the reaction zone of the process disclosed in U.S. Patent No. 7,291,761, while the molecular oxygen in the reaction zone oxidizes (burns/combusts) at least a portion of the molecular hydrogen present in the reaction gas to water vapor. This integrated process forms a product gas comprising molecular hydrogen, as well as water vapor, and dehydrogenation products. This approach requires provision and consumption of a fuel in addition to the hydrocarbon to be dehydrogenated, and the hydrogen present in the dehydrogenation product stream is known to contribute to unwanted side-reactions and commensurate decreases in the yield of the desired partial oxidation products.

Additionally, U.S. Patent No. 4,788,371 describes a process for steam dehydrogenation of hydrocarbons in the vapor phase with simultaneous oxidative reheating of the intermediate products by selective hydrogen combustion. The process utilizes a single catalyst composition, as well as steam and oxygen, to accomplish both the selective oxidation and steam dehydrogenation reactions. The particular catalysts employed comprise a Group VIII noble metal component, a Group IA and/or a Group IIA component and may contain among other modifiers a Group IIIA or IVA metal, and a halogen component. The catalytic components are supported on an inorganic substrate such as alumina. More particularly, the catalyst composition catalyzes the dehydrogenation of a hydrocarbon, which is an endothermic reaction, and also catalyzes the oxidation (combustion) of the dehydrogenation byproduct hydrogen to produce heat to sustain further oxidative dehydrogenation of the hydrocarbon. Reaction temperatures were reported as ranging between 400°C to 900°C, depending upon the particular hydrocarbon reactant involved.

Grasselli, et al., have reported successful non-oxidative dehydrogenation of light alkane hydrocarbons in combination with selective hydrogen combustion, using two different catalyst compositions which are either arranged in series of successive catalyst beds, or mixed together in a single catalyst bed. See Grasselli, et al., "Catalytic dehydrogenation (DH) of light parrafins combined with selective hydrogen combustion (SHC) I. DH -> SHC -> DH catalysts in series (co-fed process mode)," Applied Catalysis A: General 189 (1999), 1-8, and Grasselli, et al., "Catalytic dehydrogenation (DH) of light parrafins combined with selective hydrogen combustion (SHC) II. DH+SHC catalysts physically mixed (redox process mode)," Applied Catalysis A: General 189 (1999), 9-14, respectively. No oxygen was provided to the reaction stage containing the non-oxidative dehydrogenation catalyst, which comprised zeolite-supported platinum and tin, while the selective hydrogen combustion catalysts comprised an oxide of a metal selected from indium, bismuth, lead and zinc, supported on zirconium, alumina or silica substrates.

An autothermal "hybrid" process has been developed for conversion of an alkane to its corresponding alkene in a thermally integrated two-stage process which is described in U.S. Patent Application Publication No. US2008/0177117 In particular, an alkane and oxygen are provided to a first reaction stage wherein a portion of the alkane is exothermically converted, by oxidative dehydrogenation in the presence of an upstream oxidative dehydrogenation catalyst and oxygen, to form an intermediate heated product stream containing heat, a small amount of the corresponding alkene and the remaining unreacted alkane. This intermediate heated product stream is then provided to a second stage wherein the remaining unreacted alkane is converted in an endothermic non-oxidative dehydrogenation reaction in the presence of a catalyst and a weak oxidant, such as carbon dioxide, to form a cumulative product stream containing additional amounts of the corresponding alkene, as well as carbon oxides, water and hydrogen. If one or more inert/diluent materials such as nitrogen, carbon oxides, noble gases and water vapor are provided to the exothermic first stage, these will also be present in the cumulative product stream. As already mentioned, the hydrogen present in the endothermic second stage product stream is known to contribute to unwanted side-reactions and commensurate decreases in the yield of the desired partial oxidation products.

As discussed in the background section of U.S. Patent Application Publication No. US2008/0177117, chromium-based catalysts are useful for catalytic conversion of one or more $C_2$-$C_4$ alkanes to form the corresponding $C_2$-$C_4$ alkenes and hydrogen in the presence of a soft oxidant, e.g., carbon dioxide, in the absence of oxygen (i.e., "soft oxidant conversion catalysts"). Furthermore, experimental testing of the activities of various metal oxide catalysts (Cr, Ga, Ni, V, Fe, Mn and Co) supported on various support materials found that the Cr-based catalyst, supported on silica, provided superior results for conversion of propane, in the presence of carbon dioxide, to form propene. Unfortunately, it has been found that water present in the intermediate heated product stream irreversibly deactivates the chromium-based catalysts in the endothermic second stage.

Accordingly, notwithstanding the work conducted to date in this field, industry continues to grapple with the aforesaid problems of increasing overall production of alkene (i.e., increasing alkene selectivity and yield), while minimizing the costs of converting lower alkanes to their corresponding alkenes. Development of an improved process and catalyst system for converting an alkane to its corresponding alkene, which provide improved selectivity and yield of the desired product alkene and address the foregoing issues presented by existing technology, would be welcomed by industry. It is believed that the integrated processes of the present invention address these needs.

## Summary of the invention

The present invention provides a process for catalytic conversion of a $C_2$-$C_4$ alkane to its corresponding $C_2$-$C_4$ alkene. This process comprises an endothermic first step of (A) converting a $C_2$-$C_4$ alkane to its corresponding $C_2$-$C_4$ alkene by providing a $C_2$-$C_4$ alkane, a weak oxidant and heat to an endothermic reaction zone comprising an upstream catalyst to produce an intermediate product gas comprising at least the corresponding $C_2$-$C_4$ alkene and hydrogen. The weak oxidend is selected from the group consisting of steam or carbon dioxide. The next step in the process is B) exothermically converting at least a portion of the hydrogen in the intermediate product gas to water by providing the intermediate product gas and oxygen to an exothermic reaction zone to produce a cumulative product gas which comprises the corresponding $C_2$-$C_4$ alkene, water, carbon oxides and heat. The process further comprises the step of C) separating and recovering at least a portion of the heat from the cumulative product gas and providing the recovered heat to the endothermic reaction zone. At least a portion of the heat provided in step A) comprises the recovered heat and a cooled cumulative product gas is produced which comprises at least the corresponding $C_2$-$C_4$ alkene. The weak oxidant may be carbon dioxide. The cooled cumulative product gas preferably comprises no more than 5% by weight of hydrogen, based on the total weight of the cumulative product gas.

In one embodiment, the upstream catalyst is a soft oxidant conversion catalyst which catalyzes the endothermic conversion of the $C_2$-$C_4$ alkane to the corresponding $C_2$-$C_4$ alkene and hydrogen in the presence of the weak oxidant, and the downstream catalyst is a selective hydrogen combustion catalyst which catalyzes the exothermic conversion of hydrogen to water in the presence of oxygen.

The soft oxidant conversion catalyst may comprise chromium or chromium oxide; optionally, one or more metals selected from the group consisting of Mo, W, V, Ga, MG, Ni and Fe; and, optionally, one or more metals selected from the group consisting of Ag, V and Ga. The soft oxidant conversion catalyst may further comprises a support material selected from the group consisting of: alumina, titania, zirconia, silica, zeolites, rare earth metal oxides, mixed metal oxides, mesoporous materials, refractory materials, and combinations thereof. In a particular embodiment, the soft oxidant conversion catalyst may comprise, as essential materials, chromium oxide, and at least one metal selected from the group consisting of: silver and vanadium, all supported on silica or alumina.

The selective hydrogen combustion catalyst may be at least one catalyst composition selected from the group consisting of: A) a catalyst comprising a noble metal selected from the group consisting of platinum and palladium, and, optionally, another metal selected from the group consisting of tin and iridium; and B) a catalyst comprising an oxide of indium or bismuth, and, optionally, another metal selected from the group consisting of molybdenum. The selective hydrogen combustion catalyst may further comprise a support material selected from the group consisting of: alumina, titanium, zirconium, silica and zeolites, and combinations thereof.

In another embodiment, the $C_2$-$C_4$ alkane comprises propane, the corresponding $C_2$-$C_4$ alkene comprises propene, and the weak oxidant comprises carbon dioxide.

## Brief Description of the Drawings

A more complete understanding of the present invention will be gained from the embodiments discussed hereinafter and with reference to the accompanying drawing, wherein:

Figure 1 is a schematic representation of one embodiment of the process of the present invention.
Figure 2 is a schematic representation of an embodiment of the process of the present invention.

## Detailed Description of the Invention

The following definitions and meanings are provided for clarity and will be used hereinafter.

The term "hydrocarbon" means a compound which comprises at least one carbon atom and at least one hydrogen atom.

As used herein, the term "$C_2$ to $C_4$ alkane" means a straight chain or branched chain alkane having from 2 to 4 carbons atoms per alkane molecule, for example, ethane, propane and butane, which are typically in the vapor phase at ordinary temperatures and pressures (e.g., at least 10°C and 1 atmosphere). Accordingly, the term "$C_2$ to $C_4$ alkene" means a straight chain or branched chain alkene having from 2 to 4 carbons atoms per alkene molecule, for example, ethene, propene and butene.

The term "corresponding $C_2$-$C_4$ alkene" means the alkene having the same number of carbon atoms per alkene molecule as the particular $C_2$-$C_4$ alkane under discussion.

Furthermore, as used herein, the term "$C_2$ to $C_4$ alkanes and alkenes" includes at least one of the aforesaid $C_2$-$C_4$ alkanes, as well as its corresponding $C_2$-$C_4$ alkene. Similarly, when used herein in conjunction with the terms "$C_2$ to $C_4$ alkane", or "$C_2$ to $C_4$ alkene", or "$C_2$ to $C_4$ alkanes and alkenes", the terminology "a mixture thereof," means a mixture that includes at least one of the aforesaid alkanes having from 2 to 4 carbons atoms per alkane molecule, and the alkene having the same number of carbons atoms per alkene molecule as the alkane under discussion, for example, without limitation, a mixture of propane and propene, or a mixture of n-butane and n-butene.

An "inert" material, sometimes also referred to as a "diluent," is any material which is substantially inert, i.e., does not participate in, is unaffected by, and/or is inactive, in the particular reaction of concern. For example, nitrogen is generally considered to be inert in reactions that convert alkanes to their corresponding alkenes. As a more specific example, nitrogen is inert in dehydrogenation reactions that produce propene from propane. In the context of catalysts, where a mixed metal oxide catalyst useful in oxidation reactions is supported by a zirconium-based material, the zirconium-based material is considered to be inert and, as such, is understood to not directly affect, and not be directly affected by, the oxidation reaction being catalyzed by the mixed metal oxide catalyst. (Rather, without being bound by theory, it is believed that some support materials, such as zirconium, directly interact with the catalyst, which in turn may affect the conversion, selectivity, etc., of the oxidation reaction.)

The efficacy of chemical reaction processes, including those discussed herein, may be characterized and analyzed using the terms "feed conversion," "selectivity" to a particular product, and "product yield." These terms are used hereinafter and will have the following standard meanings.

The feed conversion, or simply "conversion", is the percentage of the total moles of feed (e.g., $C_3$ to $C_5$ alkanes and alkenes, such as propane and propene, or a mixture thereof) that have been consumed by the reaction, regardless of what particular products were produced, and is generally calculated as follows:

$$\text{feed conversion (\%)} = \frac{\text{moles of feed converted}}{\text{moles of feed supplied}} \times 100$$

The selectivity to a particular product, or simply "selectivity," is the percentage of the percentage of the total moles of feed (e.g., $C_3$ to $C_5$ alkanes, such as ethane, propane, and propene, or a mixture thereof) that have been consumed by the reaction, i.e., the portion of the feed that has been consumed was actually converted to the desired product, regardless of other products. Selectivity is generally calculated as follows:

$$\text{selectivity (\%)} = \frac{\text{moles of desired product produced}}{\text{moles of feed converted}} \times \frac{\text{number of carbon atoms in product}}{\text{number of carbon atoms in feed}} \times 100$$

The product yield, or simply "yield," is the percentage of the theoretical total moles of the desired product (alkene) that would have been formed if all of the feed had been converted to that product (as opposed to unwanted side products, e.g. acetic acid and $CO_x$ compounds), and is generally calculated as follows:

$$\text{product yield (\%)} = \frac{\text{moles of product produced}}{\text{moles of feed supplied}} \times \frac{\text{number of carbon atoms in product}}{\text{number of carbon atoms in feed}} \times 100$$

The term "oxygen-containing gas," as used herein, means any gas comprising from 0.01% up to 100% oxygen or oxygen-containing compounds, including for example, without limitation: air, oxygen-enriched air, nitrous oxide, nitrogen dioxide, pure oxygen, mixtures of pure oxygen or oxygen-containing compounds with at least one inert gas, such as nitrogen, and mixtures thereof. Although the oxygen containing gas may be pure oxygen gas, it is usually more economical to use an oxygen containing gas, such as air, when purity is not particularly required.

"Dehydrogenation," as used herein, means a chemical reaction in which one or more hydrogen atoms are eliminated from a hydrocarbon having at least 2 carbon atoms. Dehydrogenation is used, for example, to convert alkanes (such as, ethane, propane, and butane) into olefins (such as ethylene, propylene, and butenes, respectively). Molecular hydrogen is often a product of dehydrogenation reactions, along with the desired olefin product. In particular, "oxidative dehydrogenation" means the dehydrogenation of a hydrocarbon having at least 2 carbon atoms in the presence of oxygen and accompanied by the production of heat.

"Selective hydrogen combustion," as used herein, means a chemical process which converts hydrogen, in the presence of oxygen, to produce water and heat.

Generally, "soft oxidant conversion," as used hereinafter, means a chemical reaction in which one or more hydrogen atoms are eliminated from a hydrocarbon having at least 2 carbon atoms, and which consumes heat. Thus, soft oxidant conversion reactions require heat to be supplied from a source external to the non-oxidative dehydrogenation reaction. Since hydrogen is removed from the hydrocarbon, soft oxidant conversion may also be referred to as "non-oxidative dehydrogenation." More particularly, as used herein, soft oxidant conversion refers to the catalytic conversion of a $C_2$-$C_4$ alkane to its corresponding $C_2$-$C_4$ alkene in the presence of a soft oxidant, such as carbon dioxide, and a selective hydrogen combustion catalyst.

As used hereinafter, a "soft oxidant conversion catalyst" is a catalyst composition which catalyzes catalytic conversion of one or more $C_2$-$C_4$ alkanes to form the corresponding $C_2$-$C_4$ alkenes and hydrogen in the presence of a soft oxidant, e.g., carbon dioxide, in the absence of oxygen.

The terms "cumulatively convert" and "cumulatively produce" are each used interchangeably to describe the desired end product(s) of a set of two or more chemical reactions relative to the initial starting materials, regardless of intermediate reaction mechanisms and products other than those intended.

Endpoints of ranges are considered to be definite and are recognized to incorporate within their tolerance other values within the knowledge of persons of ordinary skill in the art, including, but not limited to, those which are insignificantly different from the respective endpoint as related to this invention (in other words, endpoints are to be construed to incorporate values "about" or "close" or "near" to each respective endpoint). The range and ratio limits, recited herein, are combinable. For example, if ranges of 1-20 and 5-15 are recited for a particular parameter, it is understood that ranges of 1-5, 1-15, 5-20, or 15-20 are also contemplated and encompassed thereby.

The present invention provides a thermally integrated process for catalytic conversion of a $C_2$-$C_4$ alkane to its corresponding $C_2$-$C_4$ alkene. The product $C_2$-$C_4$ alkene may then be further converted by catalytic partial oxidation to oxidation products, including unsaturated carboxylic acids and unsaturated nitriles. More particularly, the inventive process employs an upstream catalytic soft oxidant conversion reaction which converts a $C_2$-$C_4$ alkane to an intermediate product gas comprising the corresponding $C_2$-$C_4$ alkene and hydrogen, which is then provided to a downstream selective hydrogen combustion reaction which eliminates at least a portion of the hydrogen from the intermediate product gas and produces water and heat. The heat from the selective hydrogen combustion is recovered and provided to the upstream soft oxidant conversion reaction. A substantially hydrogen-free $C_2$-$C_4$ alkene product, i.e., comprising not more than 5% by weight hydrogen, based on the total weight of the alkene product, is produced and is suitable for providing directly to further processing steps, such as partial oxidation to unsaturated carboxylic acids or nitriles.

Catalysts suitable for use as upstream catalysts in the endothermic reaction zone are not particularly limited and include, but are not limited to, soft oxidant conversion catalysts recommended in the prior art for heterogeneously catalyzed partial dehydrogenation of hydrocarbons, in the gas phase and in the presence of a weak oxidant, to form molecular hydrogen.

Catalysts suitable for use as downstream, catalysts in the exothermic reaction zone are not particularly limited and include, but are not limited to, selective hydrogen combustion catalysts recommended in the prior art for selective combustion of hydrogen, in the gas phase and in the presence of oxygen, to form water and heat.

The upstream and downstream catalysts may be prepared by any suitable method known in the art, now or in the future. For example, the catalyst can be prepared by incipient wetness impregnation, chemical vapor deposition, hydrothermal synthesis, salt melt method, co-precipitation, and other methods. As will be discussed in further detail hereinafter, catalysts which are active for exothermic or endothermic conversion of $C_2$-$C_4$ alkanes to produce the corresponding $C_2$-$C_4$ alkenes typically comprise one or more metals and/or metal oxides. In addition, either or both of the upstream and downstream catalysts may be promoted, for example, with suitable metals or metal oxides.

Furthermore, either or both of the upstream and downstream catalysts may further comprise support material. The catalyst materials may be applied to the support by any method known in the art and at any time including, but not limited to, during preparation of the catalyst material, before or after calcination, and even before or after addition of a promoter. Typical and suitable support materials include, but are not limited to: magnesium oxide, zirconia, stabilized zirconia, stabilized alumina, yttrium stabilized zirconia, calcium stabilized zirconia, alumina, titania, silica, magnesia, nitrides, silicon carbide, cordierite, cordierite-alpha alumina, alumina-silica magnesia, zircon silicate, magnesium silicates, calcium oxide, silica-alumina, alumina-zirconia, alumina-ceria, and combinations thereof. Additionally, suitable catalyst supports may comprise rare earth metal oxides, mixed metal oxides, mesoporous materials, refractory materials, and combinations thereof. The support may be modified, stabilized, or pretreated in order to achieve the proper structural stability desired for sustaining the operating conditions under which the catalysts will be used.

The support can be in the shape of wire gauzes, monoliths, particles, honeycombs, rings, and others. Where the support is in the form of particles, the shape of the particles is not particularly limited and may include granules, beads, pills, pellets, cylinders, trilobes, spheres, irregular shapes, etc.

Monoliths typically comprise any unitary piece of material of continuous manufacture, such as, for example, pieces of metal or metal oxide, foam materials, or honeycomb structures. It is known in the art that, if desired, a reaction zone may comprise two or more such catalyst monoliths stacked upon one another. For example, the catalyst can be structured as, or supported on, a refractory oxide "honeycomb" straight channel extrudate or monolith, made of cordierite or mullite, or other configuration having longitudinal channels or passageways permitting high space velocities with a minimal pressure drop.

Furthermore, the catalyst material may be deposited as washcoats on the monolithic support by methods known to people skilled in the art. Additionally, catalyst material may be combined with the monolithic support by depositing the support material as washcoats and, successively, impregnating the support material washcoats with the active catalyst material, such as, without limitation, chromium oxide or vanadium oxide, followed by calcination of the combined support and catalyst materials.

Monolithic supports may comprise stabilized zirconia (PSZ) foam (stabilized with Mg, Ca or Y), or foams of silica, $\alpha$-alumina, cordierite, ceramics, titania, mullite, zirconium-stabilized $\alpha$-alumina, or mixtures thereof. Monolithic supports may also be fabricated from metals and their alloys, such as, for example, aluminum, steel, fecralloy, hastalloy, and others known to persons skilled in the art. Additionally, other refractory foam and non-foam monoliths may serve as satisfactory supports. The promoter metal precursor and any base metal precursor, with or without a ceramic oxide support forming component, may be extruded to prepare a three-dimensional form or structure such as a honeycomb, foam or other suitable tortuous-path or straight-path structure.

In an exemplary embodiment, the upstream catalyst should be a soft oxidant conversion catalyst which catalyzes the endothermic partial dehydrogenation of a $C_2$-$C_4$ alkane, in the presence of a mild oxidant and in the absence of oxygen, to the corresponding $C_2$-$C_4$ alkene and hydrogen. The mild oxidant may be, for example, without limitation, carbon dioxide, steam, or a combination thereof. As discussed hereinabove, many such soft oxidant conversion catalysts are known and would be suitable for use in the endothermic reaction zone in accordance with process of the present invention.

Persons of ordinary skill will be familiar with various soft oxidant conversion catalysts that may be successfully used in the endothermic reaction zone, in accordance with process of the present invention. Suitable categories of soft oxidant conversion catalysts include, but are not limited to: chromium-based catalysts, which may also comprise oxides of at least one metal selected from the group consisting of, for example, Mo, W, V, Ga, Mg, Ni, and Fe; as well as vanadium oxide-based catalysts, which may be promoted with Cr, Li, Na, K or Mg. For example, chromium-based catalysts which also comprise a metal selected from the group consisting of silver, vanadium and gallium, and which are supported on silica or alumina, are known to be particularly suitable soft oxidant conversion catalysts for use in the process of the present invention.

Also in an exemplary embodiment, the downstream catalyst should be a selective hydrogen combustion catalyst which catalyzes the exothermic combustion of hydrogen, in the presence of oxygen, to form water and heat. As discussed hereinabove, many such exothermic selective hydrogen combustion catalysts are known and would be suitable for use the exothermic reaction zone in accordance with process of the present invention. For example, supported platinum-based catalysts would be suitable for use in the exothermic reaction zone of the present invention.

Persons of ordinary skill will be familiar with various exothermic selective hydrogen combustion catalysts that may be successfully used in the exothermic reaction zone, in accordance with process of the present invention. Suitable categories of exothermic selective hydrogen combustion catalysts include, but are not limited to: oxides of metals such as indium, bismuth, lead and zinc, and catalysts comprising one or more Group VIII noble metals (such as platinum, palladium, iridium, rhodium, osmium and ruthenium) with one or more of rubidium, cesium, potassium, sodium, lithium and francium, as well as one or more of boron, gallium, indium, germanium, tin and lead. The selective hydrogen combustion catalyst may be supported on materials such as alumina, silica, zirconia, zeolites, other metal oxides, microporous materials, mesoporous materials, and refractory materials.

As will be easily recognized by skilled persons, there are many catalyst compositions suitable for use in the exothermic reaction zone in accordance with the present invention. For example, the exothermic selective hydrogen combustion catalyst may comprise platinum, supported on silica, with or without tin or indium. Another suitable selective hydrogen combustion catalyst would comprise a noble metal component comprising platinum or palladium, another component comprising tin and/or indium, and still another component comprising cesium and/or potassium, all supported on alumina or silica.

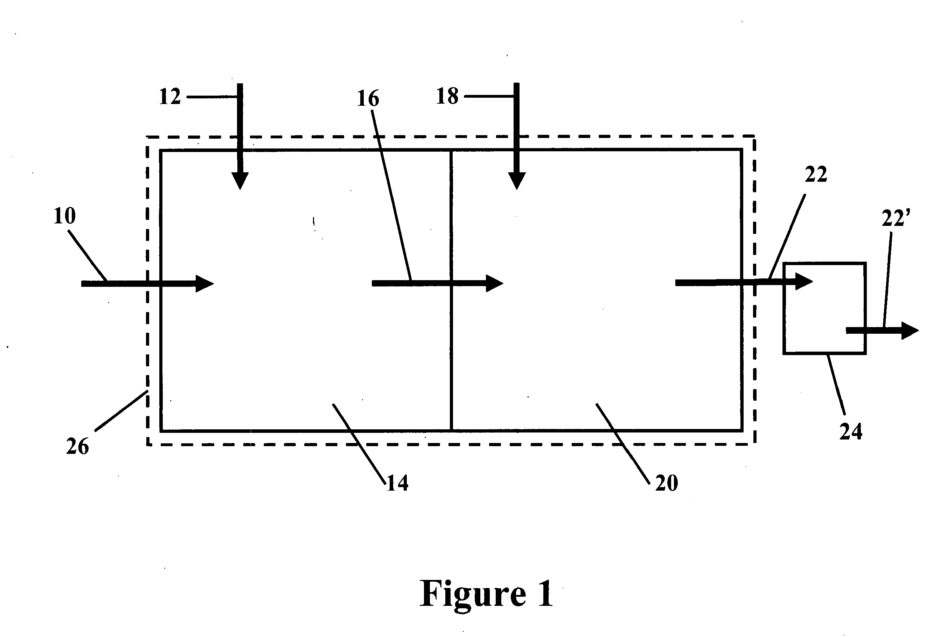

Referring now to the schematic representation of the process of the present invention provided in Figure 1, generally, a $C_2$-$C_4$ alkane 10 and a weak or mild oxidant 12, such as carbon dioxide, are contacted with an upstream catalyst (not shown per se) in an endothermic reaction zone 14 to produce an intermediate product gas 16.

The upstream catalyst is catalytically active for the endothermic (soft oxidant) conversion of the $C_2$-$C_4$ alkane 10 to its corresponding $C_2$-$C_4$ alkene. Carbon dioxide 12 may be supplied to the endothermic reaction zone 14 in any manner known to persons of ordinary skill in the art. For example, as shown in Figure 1, carbon dioxide 12 may be provided as a separate stream directly to the endothermic reaction zone 14, simultaneously with the $C_2$-$C_4$ alkane 10. Other options, which are not shown here, include, but are not limited to: blending the carbon dioxide 12 with the $C_2$-$C_4$ alkane 10 before entry into the endothermic reaction zone 14, or blending the carbon dioxide 12 with one or more other feed streams to the endothermic reaction zone 14.

One or more inert materials, or diluents (not shown), may also be provided to the endothermic reaction zone 14, separately or mixed with either, or both, of the $C_2$-$C_4$ alkane 10 and carbon dioxide 12. Suitable diluents include, but are not limited to nitrogen, noble gases and steam. The feed composition to the endothermic reaction zone 14 may be, for example, 10-80vol% $C_2$-$C_4$ alkane, 10-50vol% carbon dioxide, and the remainder nitrogen, based upon the total volume of the feed materials. Another example of a suitable feed composition for the endothermic reaction zone may be, without limitation, 30-60vol% $C_2$-$C_4$ alkane, 20-50vol% carbon dioxide, and the remainder nitrogen, based upon the total volume of the feed materials.

Suitable operating conditions for endothermic soft oxidant conversion of a $C_2$-$C_4$ alkane are generally known by persons of ordinary skill and are applicable to operation of the endothermic reaction zone. For example, carbon dioxide, the heated mixed product gas comprising unreacted $C_2$-$C_4$ alkane and, optionally, a diluent, may be supplied to the endothermic reaction zone, separately or mixed, at a total gas hourly space velocity (GHSV) of about 500 hr$^{-1}$ to 100,000 hr$^{-1}$. The reaction pressure is typically in the range of from 0.1 to about 10 atm, for example, from 0.8 to 5.0 atm, and the reaction temperature is typically maintained between 300°C and 900°C, for example, between 450°C and 700°C. Contact time between the reactants and catalyst is typically in the range of from 36 ms (100,000 h$^{-1}$) to 7.2 seconds (500 h$^{-1}$), such as, for example, from 200 ms to 5 seconds. The molecular ratio of unreacted $C_2$-$C_4$ alkane to mild oxidant, such as carbon dioxide, supplied to the exothermic reaction zone may, for example, be in a range of from 1:0.1 to 1:10, or even between 1:1 and 1:5.

At least a portion of the $C_2$-$C_4$ alkane is converted, by soft oxidant conversion in the endothermic reaction zone 14 in the presence of the soft oxidant, to produce an intermediate product gas 16 which comprises at least the corresponding $C_2$-$C_4$ alkene and hydrogen. The intermediate product gas 16 may also comprise one or more of the following compounds: unreacted $C_2$-$C_4$ alkane, oxygen, unreacted carbon dioxide, as well as other compounds including, but not limited to, carbon monoxide and water vapor.

With reference still to Figure 1, the process of the present invention further comprises contacting the intermediate product gas 16 and oxygen 18, with a downstream catalyst (not shown per se) in an exothermic reaction zone 20. The oxygen 18 may be provided in the form of an oxygen-containing gas, and it conveniently provided in the form of air.

The downstream catalyst is catalytically active for the selective combustion of hydrogen to form water and heat. Oxygen 18 may be supplied to the exothermic reaction zone 20 in any manner known to persons of ordinary skill in the art. For example, as shown in Figure 1, oxygen 18 may be provided as a separate stream directly to the exothermic reaction zone 20, simultaneously with the intermediate product gas 16. As another option, not shown here, the oxygen 18 may be blended with the intermediate product gas 16 before entry into the exothermic reaction zone 20.

One or more inert materials, or diluents (not shown), may also be provided to the exothermic (SHC) reaction zone 20, separately or mixed with either, or both, of the intermediate product gas 16 and oxygen 18. Suitable diluents include, but are not limited to nitrogen, noble gases and steam. The feed composition to the exothermic reaction zone 20 may be, for example, 10-30 vol% $C_2$-$C_4$ alkene, 0-40 vol% unreacted $C_2$-$C_4$ alkane, 10-60 vol% carbon dioxide, 1-15 vol% oxygen, and the remainder nitrogen, based upon the total volume of the feed materials. Another example of a suitable feed composition for the exothermic reaction zone 20 may be, without limitation, 5-20 vol% $C_2$-$C_4$ alkene, 0-30 vol% unreacted $C_2$-$C_4$ alkane, 30-60 vol% carbon dioxide, 5-15 vol% oxygen, and the remainder nitrogen, based upon the total volume of the feed materials.

At least a portion of the hydrogen in the intermediate product gas 16 (i.e., the hydrogen formed during dehydrogenation of the $C_2$-$C_4$ alkane) is converted (i.e., combusted, oxidized), in the exothermic reaction zone 20, to produce a cumulative product gas 22 comprising at least the corresponding $C_2$-$C_4$ alkene, water and heat. The cumulative product stream 22 may also comprise one or more of the following compounds: unreacted $C_2$-$C_4$ alkane, unreacted oxygen, unreacted hydrogen, unreacted carbon dioxide, as well as other compounds including, but not limited to, carbon monoxide and nitrogen.

Suitable operating conditions for exothermic selective hydrogen combustion are generally known by persons of ordinary skill and are applicable to operation of the exothermic reaction zone 20. For example, oxygen, the intermediate product gas and, optionally, a diluent, may be supplied to the exothermic reaction zone, separately or mixed, at a total gas hourly space velocity (GHSV) of about 1,000 hr$^{-1}$ to 100,000 hr$^{-1}$. The reaction pressure suitable for the exothermic reaction zone 20 is typically in the range of from 0.1 to about 5 atm, for example, from 0.5 to 2.0 atm, and the reaction temperature is typically maintained between 100°C and 500°C. The molecular ratio of hydrogen (or $C_2$-$C_4$ alkene) to oxygen, supplied to the exothermic reaction zone may, for example, be in a range of greater than zero and less than 1.0.

At least a portion of the heat present is separated and recovered from the cumulative product stream 22 and provided to the endothermic reaction zone 16 to provide heat for the catalytic soft oxidant conversion of the $C_2$-$C_4$ alkane 10. The way that separation of the heat from the cumulative product stream 22 is accomplished is not critical to the invention and may be accomplished in any way known, now or in the future, to persons of ordinary skill in the art. For example, without limitation, one or more heat exchangers 24, such as shell & tube heat exchangers, plate & frame heat exchangers, and air-cooled heat exchangers may be employed to remove heat from the cumulative product gas 22.

Removal of heat forms a cooled cumulative product gas 22' which comprises at least the corresponding $C_2$-$C_4$ alkene and no more than about 5% by weight of hydrogen, based on the total weight of the cumulative product gas.

Although not shown in Figure 1, it will be readily recognized by persons of ordinary skill that the cooled cumulative product stream 22' may be subjected to further processing and/or participate in additional reactions. For example, the cooled cumulative product stream 22' may be supplied directly to another reaction process, such as vapor phase oxidation of the alkene to produce unsaturated carboxylic acids or nitriles. The cooled cumulative product stream 22' may be further processed to purify the desired corresponding $C_2$-$C_4$ alkene product by separating at least a portion of unreacted reactants and other compounds from the cumulative product stream 22'.

Determination of the quantities and how to supply the reactant materials ($C_2$-$C_4$ alkane 10, carbon dioxide 12, oxygen 18, etc.) to each of the endothermic and exothermic reaction zones 14, 20 is well within the ability of persons of ordinary skill in the art, based upon the knowledge generally available as well as the particular reactions, the desired products, and the catalysts selected for use in the reaction zones. For example, where carbon dioxide is expected to interfere with the performance of the selected downstream catalyst, then the carbon dioxide should be provided to the endothermic reaction zone 14 in stoichiometric amounts with the $C_2$-$C_4$ alkane.

As shown in Figure 1, the endothermic and exothermic reaction zones 14, 20 may be contained in a single reactor 26 (shown in phantom), which may be any suitable reactor known in the art including, but not limited to, a batch reactor, a stirred tank reactor, a continuous stirred tank reactor (CSTRs), a tubular reactor, a shell-and-tube heat exchanger reactor, a multiple-pass reactor, a reactor having microchannels, a short contact time reactor, a catalytic fixed bed reactor, and a reactor having a combination of the foregoing features. Each reaction zone 14, 20 may, instead, be disposed within separate reactors (not shown), and various combinations of reactors and reaction zones may be arranged. Each reaction zone 14, 20 may or may not include sub-zones (also not shown), which differ by operating temperature, or catalyst composition, or catalyst concentration, or in other ways which are known to persons of ordinary skill. Furthermore, the upstream and downstream catalysts may be configured in their respective reaction zones in any suitable arrangement including, but not limited to, a fixed bed, a fluidized bed, and a spouted bed. All such configurations are well known in the art.

As discussed in further detail hereinafter in connection with an exemplary embodiment, it is within the ability of persons of ordinary skill in the relevant art to select appropriate operating conditions for each of the exothermic and endothermic reaction zones, depending on the particular products desired and the reactions and catalysts selected to produce the desired product.

## EXAMPLE

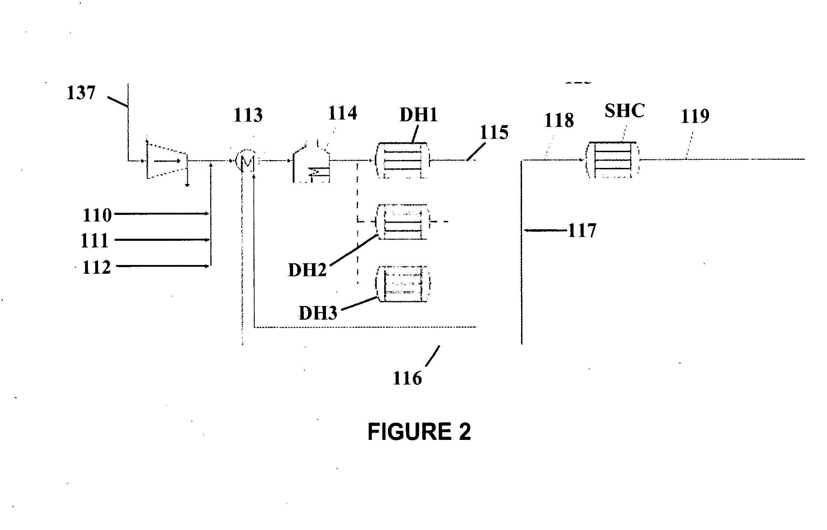

The following description refers to the schematic representation of process equipment shown in Figure 2. Three soft oxidant conversion reactors (SOC1, SOC2, SOC3), each comprising one oxidation reaction zones (not shown per se, but see Figure 1 and accompanying description above), are loaded with suitable catalysts (also not shown per se, but see Figure 1 and accompanying description above). One reactor at a time is in operation and is used to convert the propane to propene using thermally integrated soft oxidant conversion processes. When one reactor is not in use, i.e., "off-line," it undergoes catalyst regeneration. The soft oxidant conversion reactors (SOC1, SOC2, SOC3) are automatically cycled on-line and off-line by a process control system (not shown).

The fresh (i.e., not including any recycled materials) starting materials fed to the soft oxidant conversion reaction zones are: propane (110) at 13140 kg/hr, carbon dioxide (111) at 8573 kg/hr, and nitrogen (112) at 5287 kg/hr. Recycle gas (137), at 62340 kg/hr and comprising propane and carbon dioxide, is also fed to the soft oxidant conversion reaction zones, in addition to the fresh starting materials. Each of the propane, carbon dioxide, nitrogen, and recycle streams (110, 111, 112, 137) has the compositions listed in the following Table 1:

Table 1: Feed Composition to Soft Oxidant Conversion (SOC) Reaction Zones

| Component | % (by volume) | | | |
|---|---|---|---|---|
| | Propane | Nitrogen | Carbon Dioxide | Recycle |
| Propane | 100 | | | 40 |
| Nitrogen | | 100 | | |
| Carbon Dioxide | | | 100 | 60 |

In this particular application, the recycle gas (137) is compressed to 2.6 bar and combined with the propane, $CO_2$ and nitrogen (110, 111, 112). The combined propane feed stream (113) is heated by exchange with the soft oxidant conversion reactor effluent (115) to 525°C and then in a feed heater (114) to the reaction temperature, 625°C.

Each soft oxidant conversion reactor is loaded with a catalyst containing 10% $Cr_2O_3$ supported on Merck 10181 for conversion of propane in the presence of a weak oxidant (the carbon dioxide), to produce propene and hydrogen. The contact time is 0.50 sec*ml/g. Heat from the selective hydrogen combustion reactor (SHC) and heat from the regeneration step of the soft oxidant conversion reactors (SOC1, SOC2, SOC3) can be used to offset the energy requirements of the soft oxidant conversion reaction to obtain a heat neutral balance. The composition of the effluent gas (115) from the soft oxidant conversion reaction zones is provided in the following Table 2:

Table 2: Product Stream Composition from Soft Oxidant Conversion Reaction Zones

| Component | % (by volume) |
|---|---|
| Carbon Dioxide | 38 |
| Carbon Monoxide | 6 |
| Ethylene | 1 |
| Hydrogen | 7 |
| Methane | 2 |
| Nitrogen | 8 |
| Propane | 24 |
| Propene | 10 |
| Water | 6 |

To the cooled effluent gas (116) is combined with 2767 kg/hr oxygen (117). The combined stream (118) is fed to the selective hydrogen combustion reactor (SHC). The reactor (SHC) is loaded with a suitable supported platinum-based catalyst. The composition of the effluent gas (119) from the SHC is provided in the following Table 3:

Table 3: Product Stream Composition from Selective Hydrogen Combustion

| Component | % (by volume) |
|---|---|
| Carbon Dioxide | 37 |
| Carbon Monoxide | 6 |
| Ethylene | 1 |
| Hydrogen | 1 |
| Methane | 2 |
| Nitrogen | 8 |
| Propane | 23 |
| Propene | 10 |
| Water | 12 |

The effluent gas (119) may be provided to further processing and/or reaction stages, as desired, with or without additional materials such as oxygen, inerts, other hydrocarbons, etc.

**Claims**

**1.** A process for conversion of a $C_2$-$C_4$ alkane to its corresponding $C_2$-$C_4$ alkene, said process comprising the steps of:

A) converting a $C_2$-$C_4$ alkane to its corresponding $C_2$-$C_4$ alkene by providing a $C_2$-$C_4$ alkane, a weak oxidant and heat to an endothermic reaction zone comprising an upstream catalyst to produce an intermediate product gas comprising at least the corresponding $C_2$-$C_4$ alkene and hydrogen, wherein the weak oxidant is selected from the group conaisting of steam or carbon dioxide;

B) converting at least a portion of the hydrogen in the intermediate product gas to water by providing the

intermediate product gas and oxygen to an exothermic reaction zone to produce a cumulative product gas which comprises the corresponding $C_2$-$C_4$ alkene, water, carbon oxides and heat; and
C) separating and recovering at least a portion of the heat from the cumulative product gas and providing the recovered heat to the endothermic reaction zone, wherein at least a portion of the heat provided in step A) comprises the recovered heat and wherein a cooled cumulative product gas is produced which comprises at least the corresponding $C_2$-$C_4$ alkene.

2. The process of Claim 1, wherein the weak oxidant is carbon dioxide.

3. The process of Claim 1, wherein the upstream catalyst is a soft oxidant conversion catalyst which catalyzes the endothermic conversion of the $C_2$-$C_4$ alkane to the corresponding $C_2$-$C_4$ alkene in the presence of the weak oxidant.

4. The process of Claim 1, wherein the downstream catalyst is a selective hydrogen combustion catalyst which catalyzes the exothermic conversion of hydrogen to water in the presence of oxygen.

5. The process of Claim 3, wherein the soft oxidant conversion catalyst comprises:

   A) chromium or chromium oxide;
   B) optionally, one or more metals selected from the group consisting of Mo, W, V, Ga, Mg, Ni and Fe; and
   C) optionally, one or more metals selected from the group consisting of Ag, V and Ga.

6. The process of Claim 3, wherein the soft oxidant conversion catalyst comprises a support material.

7. The process of Claim 6, wherein the support material of the soft oxidant conversion catalyst comprises a material selected from the group consisting of:

   alumina, titania, zirconia, silica, zeolites, rare earth metal oxides, mixed metal oxides, mesoporous materials, refractory materials, and combinations thereof.

8. The process of Claim 7, wherein the soft oxidant conversion catalyst comprises, as essential materials, chromium oxide, and at least one metal selected from the group consisting of: silver and vanadium.

9. The process of Claim 8, wherein the support material of the soft oxidant conversion catalyst comprises silica.

10. The process of Claim 4, wherein the selective hydrogen combustion catalyst comprises at least one catalyst composition selected from the group consisting of:

    A) a catalyst comprising a noble metal selected from the group consisting of platinum and palladium, and, optionally, another metal selected from the group consisting of tin and iridium; and
    B) a catalyst comprising an oxide of indium or bismuth, and, optionally, another metal selected from the group consisting of molybdenum.

11. The process of Claim 9, wherein the selective hydrogen combustion catalyst comprises a support material.

12. The process of Claim 11, wherein the support material of the selective hydrogen combustion catalyst comprises a material selected from the group
consisting of: alumina, titanium, zirconium, silica and zeolites, and combinations thereof.

13. The process of Claim 1, wherein the $C_2$-$C_4$ alkane comprises propane, the corresponding $C_2$-$C_4$ alkene comprises propene, and the weak oxidant comprises carbon dioxide.

14. The process of Claim 1, wherein the cooled cumulative product gas further comprises no more than 5% by weight of hydrogen, based on the total weight of the cumulative product gas.

**Patentansprüche**

1. Verfahren zur Umwandlung eines $C_2$-$C_4$ Alkans in dessen entsprechendes $C_2$-$C_4$ Alken, wobei das Verfahren die Schritte umfaßt:

A) das Umwandeln eines $C_2$-$C_4$ Alkans in dessen entsprechendes $C_2$-$C_4$ Alken durch Bereitstellen eines $C_2$-$C_4$ Alkans, eines schwachen Oxidationsmittels und Wärme in einer endothermen Reaktionszone, umfassend einen stromaufwärtigen Katalysator, zur Erzeugung eines Zwischenproduktgases, umfassend mindestens das entsprechende $C_2$-$C_4$ Alken und Wasserstoff, wobei das schwache Oxidationsmittel aus der Gruppe, bestehend aus Dampf oder Kohlendioxid, ausgewählt ist,

B) das Umwandeln mindestens eines Teils des Wasserstoffs in dem Zwischenproduktgas in Wasser durch Bereitstellen des Zwischenproduktgases und Sauerstoffs in einer exothermen Reaktionszone zur Erzeugung eines kumulativen Produktgases, welches das entsprechende $C_2$-$C_4$ Alken, Wasser, Kohlendioxid und Wärme umfaßt, und

C) das Abtrennen und Rückgewinnen mindestens eines Teils der Wärme von dem kumulativen Produktgas und das Bereitstellen der rückgeführten Wärme in eine endotherme Reaktionszone, wobei mindestens ein Teil der Wärme, bereitgestellt in Schritt A), die rückgeführte Wärme umfaßt und wobei ein abgekühltes kumulatives Produktgas erzeugt wird, welches mindestens das entsprechende $C_2$-$C_4$ Alken umfaßt.

2. Verfahren gemäß Anspruch 1, wobei das schwache Oxidationsmittel Kohlendioxid ist.

3. Verfahren gemäß Anspruch 1, wobei der stromaufwärtige Katalysator ein weicher Oxidationsumwandlungskatalysator ist, welcher die endotherme Umwandlung des $C_2$-$C_4$ Alkans in das entsprechende $C_2$-$C_4$ Alken in der Gegenwart des schwachen Oxidationsmittels katalysiert.

4. Verfahren gemäß Anspruch 1, wobei der stromabwärtige Katalysator ein selektiver Wasserstoffverbrennungskatalysator ist, welcher die exotherme Umwandlung von Wasserstoff zu Wasser in der Gegenwart von Sauerstoff katalysiert.

5. Verfahren gemäß Anspruch 3, wobei der weiche Oxidationsumwandlungskatalysator umfaßt:

A) Chrom oder Chromoxid,

B) gegebenenfalls ein oder mehrere Metalle, ausgewählt aus der Gruppe, bestehend aus Mo, W, V, Ga, Mg, Ni und Fe, und

C) gegebenenfalls ein oder mehrere Metalle, ausgewählt aus der Gruppe, bestehend aus Ag, V und Ga.

6. Verfahren gemäß Anspruch 3, wobei der weiche Oxidationsumwandlungskatalysator ein Trägermaterial umfaßt.

7. Verfahren gemäß Anspruch 6, wobei das Trägermaterial des weichen Oxidationsumwandlungskatalysators ein Material umfaßt, ausgewählt aus der Gruppe, bestehend aus: Aluminiumoxid, Titanoxid, Zirkoniumoxid, Siliciumoxid, Zeolithe, Seltenerdmetalloxide, Metallmischoxide, mesoporöse Materialien, feuerfeste Materialien und Kombinationen davon.

8. Verfahren gemäß Anspruch 7, wobei der weiche Oxidationsumwandlungskatalysator als wesentliche Materialien Chromoxid und mindestens ein Metall, ausgewählt aus der Gruppe, bestehend aus: Silber und Vanadium, umfaßt.

9. Verfahren gemäß Anspruch 8, wobei das Trägermaterial des weichen Oxidationsumwandlungskatalysators Siliciumoxid umfaßt.

10. Verfahren gemäß Anspruch 4, wobei der selektive Wasserstoffverbrennungskatalysator mindestens eine Katalysatorzusammensetzung umfaßt, ausgewählt aus der Gruppe, bestehend aus:

A) einem Katalysator, umfassend ein Nobelmetall, ausgewählt aus der Gruppe, bestehend aus Platin und Palladium, und gegebenenfalls ein weiteres Metall, ausgewählt aus der Gruppe, bestehend aus Zinn und Iridium, und

B) einem Katalysator, umfassend ein Oxid von Indium oder Bismuth, und gegebenenfalls einem weiteren Metall, ausgewählt aus der Gruppe, bestehend aus Molybdän.

11. Verfahren gemäß Anspruch 9, wobei der selektive Wasserstoffverbrennungskatalysator ein Trägermaterial umfaßt.

12. Verfahren gemäß Anspruch 11, wobei das Trägermaterial des selektiven Wasserstoffverbrennungskatalysators ein Material umfaßt, ausgewählt aus der Gruppe, bestehend aus: Aluminiumoxid, Titan, Zirkonium, Siliciumoxid und Zeolithen und Kombinationen davon.

13. Verfahren gemäß Anspruch 1, wobei das $C_2$-$C_4$ Alkan Propan umfaßt, das entsprechende $C_2$-$C_4$ Alken Propen umfaßt und das schwache Oxidationsmittel Kohlendioxid umfaßt.

14. Verfahren gemäß Anspruch 1, wobei das abgekühlte kumulative Produktgas nicht mehr als 5 Gew.-% Wasserstoff, bezogen auf das Gesamtgewicht des kumulativen Produktgases, umfaßt.

**Revendications**

1. Procédé pour la conversion d'un $C_2$-$C_4$-alcane en son $C_2$-$C_4$-alcène correspondant, ledit procédé comprenant les étapes de :

   A) conversion d'un $C_2$-$C_4$-alcane en son $C_2$-$C_4$-alcène correspondant par fourniture d'un $C_2$-$C_4$-alcane, d'un oxydant faible et de chaleur à une zone de réaction endothermique comprenant un catalyseur amont pour produire un gaz de produits intermédiaires comprenant au moins le $C_2$-$C_4$-alcène correspondant et de l'hydrogène, où l'oxydant faible est choisi dans le groupe consistant en la vapeur et le dioxyde de carbone ;
   B) conversion d'au moins une partie de l'hydrogène dans le gaz de produits intermédiaires en eau par fourniture du gaz de produits intermédiaires et d'oxygène à une zone de réaction exothermique pour produire un gaz de produits cumulatifs qui comprend le $C_2$-$C_4$-alcène correspondant, de l'eau, des oxydes de carbone et de la chaleur ; et
   C) séparation et récupération d'au moins une partie de la chaleur à partir du gaz de produits cumulatifs et fourniture de la chaleur récupérée à la zone de réaction endothermique, où au moins une partie de la chaleur fournie dans l'étape A) comprend la chaleur récupérée et où un gaz de produits cumulatifs refroidi qui comprend au moins le $C_2$-$C_4$-alcène correspondant est produit.

2. Procédé selon la revendication 1 où l'oxydant faible est le dioxyde de carbone.

3. Procédé selon la revendication 1 où le catalyseur amont est un catalyseur de conversion à oxydant doux qui catalyse la conversion endothermique du $C_2$-$C_4$-alcane en le $C_2$-$C_4$-alcène correspondant en présence de l'oxydant faible.

4. Procédé selon la revendication 1 où le catalyseur aval est un catalyseur de combustion d'hydrogène sélective qui catalyse la conversion exothermique de l'hydrogène en eau en présence d'oxygène.

5. Procédé selon la revendication 3 où le catalyseur de conversion à oxydant doux comprend :

   A) du chrome ou de l'oxyde de chrome ;
   B) éventuellement un ou plusieurs métaux choisis dans le groupe consistant en Mo, W, V, Ga, Mg, Ni et Fe ; et
   C) éventuellement, un ou plusieurs métaux choisis dans le groupe consistant en Ag, V et Ga.

6. Procédé selon la revendication 3 où le catalyseur de conversion à oxydant doux comprend un matériau support.

7. Procédé selon la revendication 6 où le matériau support du catalyseur de conversion à oxydant doux comprend un matériau choisi dans le groupe consistant en : l'alumine, l'oxyde de titane, la zircone, la silice, les zéolithes, les oxydes de métaux des terres rares, les oxydes métalliques mixtes, les matériaux mésoporeux, les matériaux réfractaires et leurs combinaisons.

8. Procédé selon la revendication 7 où le catalyseur de conversion à oxydant doux comprend, comme matériaux essentiels, de l'oxyde de chrome, et au moins un métal choisi dans le groupe consistant en : l'argent et le vanadium.

9. Procédé selon la revendication 8 où le matériau support du catalyseur de conversion à oxydant doux comprend de la silice.

10. Procédé selon la revendication 4 où le catalyseur de combustion d'hydrogène sélective comprend au moins une composition de catalyseur choisie dans le groupe consistant en :

A) un catalyseur comprenant un métal noble choisi dans le groupe consistant en le platine et le palladium et, éventuellement, un autre métal choisi dans le groupe consistant en l'étain et l'iridium ; et

B) un catalyseur comprenant un oxyde d'indium ou de bismuth, et, éventuellement, un autre métal choisi dans le groupe consistant en le molybdène.

11. Procédé selon la revendication 9 où le catalyseur de combustion d'hydrogène sélective comprend un matériau support.

12. Procédé selon la revendication 11 où le matériau support du catalyseur de combustion d'hydrogène sélective comprend un matériau choisi dans le groupe consistant en : l'alumine, le titane, le zirconium, la silice et les zéolithes, et leurs combinaisons.

13. Procédé selon la revendication 1 où le $C_2$-$C_4$-alcane comprend du propane, le $C_2$-$C_4$-alcène correspondant comprend du propène, et l'oxydant faible comprend du dioxyde de carbone.

14. Procédé selon la revendication 1 où le gaz de produits cumulatifs refroidi comprend en outre pas plus de 5 % en poids d'hydrogène, sur la base du poids total du gaz de produits cumulatifs.

10 12 16 18 22 22'

26 24

14 20

**Figure 1**

EP 2 143 701 B1

FIGURE 2

EP 2 143 701 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0117146 A
- US 5705684 A
- US 7291761 B

- US 4788371 A
- US 20080177117 A

**Non-patent literature cited in the description**

- **Grasselli et al.** Catalytic dehydrogenation (DH) of light parrafins combined with selective hydrogen combustion (SHC) I. DH -> SHC -> DH catalysts in series (co-fed process mode),. *Applied Catalysis A: General,* 1999, vol. 189, 1-8

- **Grasselli et al.** Catalytic dehydrogenation (DH) of light parrafins combined with selective hydrogen combustion (SHC) II. DH+SHC catalysts physically mixed (redox process mode),. *Applied Catalysis A: General,* 1999, vol. 189, 9-14